# EUROPÄISCHE PATENTANMELDUNG

(11) **EP 2 368 507 A1**
(43) Veröffentlichungstag der Anmeldung: **28.09.2011**
(21) Anmeldenummer: 10168276.3
(22) Anmeldetag: 02.07.2010
(51) Int. Cl.: A61B 18/14

(54) **Bipolare Pinzette**

(30) Priorität: 24.03.2010 LV 100041
(71) Anmelder: OOO "Novye Energeticheskie, Moskau 107045 (RU); Golsen Limited, P.C. 3041 Limassol (CY)
(72) Erfinder: Belov, Sergey V., 119311 Moskva (RU); Ganin, Igor Y., 124498 Moskva (RU); Danileyko, 142092 Troitsk (RU); Nefedov, Sergey M., 115612 Moskva (RU); Osiko, Vjacheslav V., 117420 Moskva (RU); Salyuk, Viktor A., 117321 Moskva (RU)
(74) Vertreter: Jeck, Anton

(57) **Zusammenfassung**

Das Ziel der vorliegenden Erfindung ist es, eine elektrochirurgische bipolare Pinzette zu schaffen, die eine Verletzung des Gewebes verringert und einen erhöhten Abnutzungswiderstand aufweist. Das ist dadurch erreicht, dass die vollen Branchen aus verfestigtem Kupfer bestehen, deren Enden mit Rhodium bedeckt sind. Die punktförmige Koagulation, die mit der Pinzette gemacht wird, ermöglicht effektive neurochirurgische Operationen von vielen kleinen Gefäßen in kurzer Zeit.

## Beschreibung

Die Erfindung betrifft eine bipolare Pinzette nach dem Oberbegriff des Anspruchs 1.

Die Erfindung gehört zu den elektrochirurgischen Instrumenten und kann bei chirurgischen Operationen benutzt werden, unter anderem in der Neurochirurgie.

Bekannt ist eine bipolare zweibranchige elektrochirurgische Pinzette (USA No. 6293946). Die Branchen sind aus rostfreiem Stahl hergestellt und haben Spitzen in Form von länglichen Eisenstangen, die mit einer 0,01 - 0,05 Zoll (0,254 - 1,27 mm) dicken Silberschicht bedeckt sind. Der Nachteil dieser Erfindung besteht darin, dass dieser Überzug unbeständig ist. Bei der Arbeit wird er infolge elektrochemischer Prozesse schnell zerstört.

Am ähnlichsten ist eine bipolare elektrochirurgische Pinzette (RU 2299702 C2). Diese Pinzette besteht aus einem Körper mit zwei dort festgemachten und voneinander elektrisch isolierten Branchen. Dabei ist jede Branche mit einer Spitze verbunden, die völlig aus Metall mit einer hohen Wärmeleitfähigkeit hergestellt ist. Diese Pinzette ist dadurch gekennzeichnet, dass die Branchen auf der Seite der Spitzenbefestigung eine Öffnung und einen Schlitz haben. Die Spitzen auf der Seite der Branche haben eine Verstärkungsrippe und eine Öffnung, die mit der Öffnung der Branche fluchtet. Dabei ist in den genannten Spitzen- und Branchenöffnungen ein Element in der Form von einem Niet angeordnet. Die Verstärkungsrippe ist in dem Branchenschlitz angeordnet und eingenietet. Die Branche und die Spitze sind zusätzlich miteinander entlang der anliegenden Oberflächen verlötet. Die Nachteile dieser vorliegenden Konstruktion sind folgende:
- eine Unzuverlässigkeit der Nietverbindung der stromleitenden Branchen und der stromleitenden Spitze der Pinzette
- keine Daten über das Spitzenmaterial
- ein Aufkommen einer Kontaktspannung bei der Verbindung der verschiedenen Materialien, die zur Entstehung eines zusätzlichen Widerstands zwischen den Branchen und den Spitzen führt.

Eine spezifische Anforderung an die Neurochirurgie (Elektrochirurgie) ist ein schnelles Koagulieren von vielen kleinen Gefäßen, die sich in der Tiefe sowie auf der Oberfläche der Operationswunde befinden. Dabei darf das umliegende Gewebe nur minimal verletzt werden. Die Notwendigkeit des Koagulierens von einigen (Dutzenden von) Gefäßen in kurzer Zeit schafft zusätzliche Bedingungen für eine schnelle Wärmeableitung aus dem Arbeitsbereich. Dies fördert eine hohe Wärmeleitfähigkeit der Spitzen für eine Wärmeableitung und eine ziemlich hohe Wärmeleitfähigkeit für die Akkumulation der abgeleiteten Wärme.

Es ist Aufgabe der Erfindung, eine elektrochirurgische bipolare Pinzette zu schaffen, die eine hocheffektive Wärmeableitung von den Spitzen gewährleistet und unanbrennende Eigenschaften und einen erhöhten Abnutzungswiderstand aufweist. Die genannten Eigenschaften führen zu einer minderen Verletzung des umliegenden Gewebes während des Koagulierens und steigern die Arbeitsdauer der Pinzette.

Die gestellte Aufgabe wird durch die Merkmale des Anspruchs 1 gelöst.

Die konstruktiv bipolare elektrochirurgische Pinzette weist zwei voll isolierte Branchen auf, die aus einem stromleitenden Stoff mit einer hohen Wärmeleitfähigkeit hergestellt sind. Dadurch ist eine begleitende Wärmeverletzung des Gewebes infolge einer hocheffektiven Wärmeableitung verringert.

Die Außenflächen der distalen Spitzen sind mit Rhodium bedeckt. Es ist bekannt, dass dank einer hohen Elektroerosionsbeständigkeit Rhodium und seine Legierungen als Material für Kontakte benutzt werden und dass hinsichtlich der chemischen Beständigkeit in den meisten Korrosionsmedien Rhodium das Platin übertrifft.

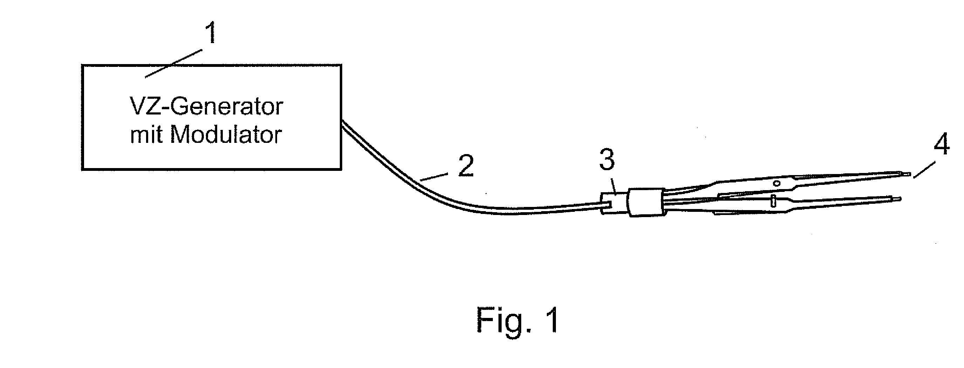

Eine Ausführung der Pinzette ist der Zeichnung entnehmbar.

Die vorliegende elektrochirurgische bipolare Pinzette funktioniert folgenderweise:

Der Strom mit einer hohen Frequenz ist über ein Kabel 2 von einem Generator 1 zu den Elektroden der Stromeinführung 3 geleitet. Diese Stromeinführungen 3 sind konstruktiv mit den proximalen Branchenenden verbunden. Dabei bilden die voneinander isolierten distalen Branchenenden des Instruments aktive bipolare Elektroden. Die Enden der Pinzette haben zwei Berührungspunkte mit dem Gewebe. Der Hochfrequenzstrom, der zum Instrument geleitet wird, fließt zwischen den Berührungspunkten der Elektroden in das Gewebe. Das Bedecken der Branchenenden 4 mit Rhodium fördert ihre Korrosionsbeständigkeit.

## Patentansprüche

1. Elektrochirurgische bipolare Pinzette mit zwei isolierten Branchen,
**dadurch gekennzeichnet,**
**dass** die Branchen aus einem Stoff mit hoher Wärmeleitfähigkeit hergestellt sind,
**dass** an den proximalen Branchenenden sich Elektroden für eine Stromeinführung (3) befinden und
**dass** die distalen Branchenenden (4) einen Korrosionsschutzüberzug aufweisen.

2. Elektrochirurgische bipolare Pinzette nach Anspruch 1,
**dadurch gekennzeichnet,**
**dass** als Material für die Branchenherstellung verfestigtes Kupfer benutzt ist.

3. Elektrochirurgische bipolare Pinzette nach Anspruch 2,
**dadurch gekennzeichnet,**
**dass** als Korrosionsschutzüberzug der Branchenenden (4) Rhodium benutzt ist.

4. Elektrochirurgische bipolare Pinzette nach Anspruch 3,
**dadurch gekennzeichnet,**
**dass** die Rhodiumschicht 0,5 bis 10 Mikrometer dick ist.

5. Elektrochirurgische bipolare Pinzette nach Anspruch 1 bis 4,
**dadurch gekennzeichnet,**
**dass** die Branchen über ein isolierendes Gelenk miteinander verbunden sind.

6. Elektrochirurgische bipolare Pinzette nach Anspruch 1 bis 5,
**dadurch gekennzeichnet,**
**dass** den Stromeinführungen (3) der Branchen HF-Strom zuführbar ist.
